# EUROPÄISCHE PATENTSCHRIFT

(11) **EP 1 592 369 B1**
(45) Veröffentlichungstag und Bekanntmachung des Hinweises auf die Patenterteilung: **08.07.2009**
(21) Anmeldenummer: 03815690.7
(22) Anmeldetag: 20.05.2003
(51) Int. Cl.: A61F 2/36

(54) **HÜFTGELENKPROTHESE MIT EINEM IN DEN OBERSCHENKELKNOCHEN EIN ZUSETZENDEN SCHAFT**
HIP JOINT PROSTHESIS COMPRISING A SHAFT TO BE INSERTED INTO THE FEMUR
PROTHESE DE LA HANCHE, MUNIE D'UNE TIGE A INSERER DANS LE FEMUR

(30) Priorität: 10.02.2003 US 361007
(43) Veröffentlichungstag der Anmeldung: 09.11.2005
(73) Patentinhaber: WALDEMAR LINK GmbH & Co. KG, 22339 Hamburg (DE)
(72) Erfinder: LINK, Helmut, D., 22397 Hamburg (DE)
(74) Vertreter: Glawe, Delfs, Moll
(86) Internationale Anmeldenummer: PCT/EP2003/005292
(87) Internationale Veröffentlichungsnummer: WO 2004/069102

(56) Entgegenhaltungen:
- EP-A- 0 093 230
- EP-A- 0 128 036
- EP-A- 0 761 182
- EP-A- 1 044 665
- WO-A-93/08771
- WO-A-99/37241
- DE-A- 3 406 358
- DE-A- 19 740 755
- FR-A- 2 194 123
- FR-A- 2 356 465
- FR-A- 2 703 583
- FR-A- 2 728 160
- US-A- 3 995 323
- US-A- 5 370 698
- US-A- 5 658 349
- US-A1- 2002 127 261

## Beschreibung

Das spongiöse Knochengewebe in der Metaphyse des Oberschenkelknochens weist eine komplizierte Struktur aus Knochenbälkchen auf, die die druck- und zugbelasteten Teile des Knochens am Schenkelhals, dem großen Trochanter, dem kleinen Trochanter und der Diaphyse druck- und zugübertragend verbinden. In ihrer Summe bilden sie durchgehende Zug- und Druck-Trajektorien (Farbatlanten der Medizin, Bd 7: Bewegungsapparat I. Verlag Thieme, Stuttgart, 1992). Wenn der Schaft einer Hüftgelenkprothese eingesetzt wird, werden insbesondere die primären Zugtrajektorien, die den Schenkelhals mit dem gegenüberliegenden, intertrochantären Oberflächenbereich des Knochens verbinden, großenteils unterbrochen. Wenn sie anschließend nicht mehr an der Kraftübertragung beteiligt sind, bilden sie sich zurück. Dies gilt insbesondere bei Verwendung solcher Prothesen, deren Prothesenschaft in der Diaphyse verklemmt wird und bei denen der proximale, metaphysäre Bereich des Oberschenkelknochens besonders in seinem lateralen Teil kaum in die Kraftübertragung einbezogen wird. Man hat versucht, durch sogenannte Zuganker den Prothesenschaft mit dem Bereich des großen Trochanters zu verbinden und diesen dadurch in den Kräftefluß einzubeziehen. Dabei wurde eine mit dem Prothesenschaft verbundene Stange durch den großen Trochanter geführt und außen mit einer Kontermutter versehen, so daß bei Belastung der Hüftprothese ein Zug auf den großen Trochanter ausgeübt wird (US-A-3,995,323, EP-B-93230, DE-B-1943598). Es hat sich aber gezeigt, daß derartige mechanische Zuganker in Folge der ständigen Wechselbelastung schnell locker werden und deshalb nur kurzfristig wirken. Ferner ist es bekannt, den Schaft oder einen davon lateral in den Bereich des großen Trochanters vorragenden Flügel so auszubilden, daß sich eine innige Verbindung mit der in Poren oder Öffnungen dieses Flügels einwachsenden Knochensubstanz ergibt (GB-A-1030145, FR-A-2356465, EP-A-128036, EP-A-222236, EP-A-95440, EP-B-601223, EP-A-1044665, US-A-5370698, FR-C-2194123). Um die Verbindung des Knochens mit der Prothesenoberfläche zu fördern, ist es auch bekannt, die Prothesenoberfläche osteokonduktiv zu gestalten. So bezeichnet man Oberflächen, die das benachbarte Knochenwachstum dulden. Dazu gehören Oberflächen aus Titanlegierungen sowie Beschichtungen, die Kalziumphosphat oder Hydroxylapatit enthalten (EP-A-761182, WO9308771).

In jüngerer Zeit sind Substanzen bekannt geworden, die das Knochenwachstum nicht nur wie die osteokonduktiven Oberflächen dulden, sondern undifferenzierte pluripotente Stammzellen zur Umbildung in Knochenzellen stimulieren (Albrechtsson, Johansson: Osteoinduction, Osteoconduction and Osseointegration; In: Gunzburg Hrsg.: The use ob bone substitutes in Spine Surgery; Springer. - Denissen, H. et al.: Ceramic hydroxyapatite Implants for the Release of Bisphosphonate; in: Bone and Mineral 1994, S. 123-134. - Yoshinari, M. et al.: Bone response sto calcium phosphate-coated and bisphosphonate-immobilized titanium implants; in: Biomaterials 2002 S. 2879-2885. - Yoshinari, M. et al.: Immobilization of bisphosphonates on surface modified titanium; in: Biomaterials 2001 S. 709-715). Zu diesen Substanzen gehören Bisphosphonate und Bone Morphogenic Proteins (kurz: BMP). Diese kann man auch zur Ausrüstung der Oberflächen von Knochenprothesen, einschließlich Hüftprothesen, verwenden (US-A-2002/0127261). Sie führen zu einer sehr innigen Verbindung der Prothesenoberfläche mit dem Knochen, die im Falle einer Reoperation unerwünscht sein kann, weil dadurch die Lösung der zu entfernenden Prothese vom Knochen behindert werden kann.

Der Erfindung liegt die Aufgabe zugrunde, die Einbindung einer Oberschenkel-Hüftprothese in den Knochen zu verbessern, ohne die Reoperierbarkeit zu gefährden. Die erfindungsgemäße Lösung besteht in den Merkmalen der Ansprüche.

Demnach ist vorgesehen, daß bei einer Hüftgelenkprothese mit einem in den Oberschenkelknochen einzusetzenden Schaft ausschließlich die im trochantären Bereich zu liegen bestimmte Oberfläche der Prothese oder ein Teil dieser Oberfläche mit der osteoinduktiven Substanz ausgerüstet wird.

Wenn die trochantäre Oberfläche der Prothese einen vom Schaft in den trochantären Bereich vorragenden Vorsprung umfaßt, ist zweckmäßigerweise ausschließlich die Oberfläche dieses Vorsprungs oder ein Teil derselben mit der osteoinduktiven Substanz ausgerüstet.

Besonders zweckmäßig ist es wenn die Substanz in eine Beschichtung eingebracht wird, die außerdem porös sein sollte. Die Beschichtung kann von beliebiger Art sein. Beispielsweise kann es sich um einen poröse Metallschicht handeln. Besonders vorteilhaft sind solche Beschichtungen, die von Haus aus osteokonduktiv sind und beispielsweise aus Kalziumphosphat oder Hydroxylapatit bestehen.

Die Wirkung der Erfindung besteht darin, daß sehr rasch nach der Operation Knochenzellen in unmittelbarer Nachbarschaft und Verbindung zur Prothesenoberfläche entstehen. Damit wird erreicht, daß sich nicht infolge Relativbewegungen zwischen der Knochenoberfläche und dem Knochen zunächst ein Spalt oder eine bindegewebige Zwischenschicht bildet, die den späteren innigen Verbund beeinträchtigt oder unmöglich macht. Dank der Erfindung findet an der trochantären Oberfläche der Prothese eine raschere Knochenanlagerung und ein Knocheneinbau in deren Poren und Vertiefungen statt, so daß der trochantäre Knochenbereich rasch eine dauerhafte Verbindung mit der Prothese erhält und als Folge davon an der Kraftübertragung beteiligt wird.

Die erfindungsgemäße Maßnahme ist nur für den trochantären Bereich der Prothese vorgesehen. Osteoinduktiv modifizierte Oberflächen auch in anderen Prothesenbereichen einzusetzen, ist nicht erwünscht, weil dadurch die Lösung der Prothese vom Knochen im Falle einer Reoperation wegen deren inniger Verbindung behindert werden kann.

Der die osteoinduktive Substanz enthaltende Teil der Oberfläche des trochantären Prothesenteils weist zweckmäßigerweise Poren oder Hinterschnitte in bezug auf die Lateralrichtung auf, damit die sich aufgrund der Osteoinduktion gebildete Knochensubstanz nicht nur durch Haftung an der Oberfläche sondern auch durch Formschluß daran verankern kann.

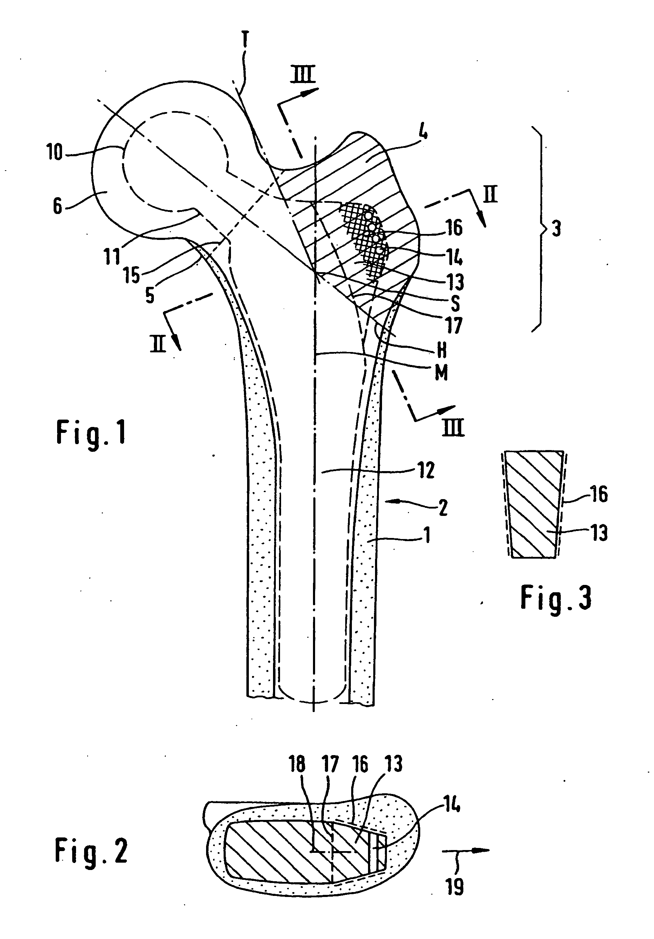

Als trochantäre Oberfläche der Prothese ist diejenige anzusehen, die dazu bestimmt ist, nach normaler Implantation innerhalb des trochantären Bereichs des Oberschenkelknochens zu liegen. Der trochantäre Teil des Knochens ist der in Fig. 1 schraffierte Teil. Geht man vom Schnittpunkt zwischen der Schenkelhals-Mittellinie mit der Mittellinie des proximalen Diaphysen-Endes aus, so liegt der trochantäre Bereich lateral von der von diesem Schnittpunkt gezogenen Tangente an den oberen Rand des Hüftkopfs und lateral von den diese Tangente fortsetzenden Teil der Schenkelhals-Mittellinie. Die trochantäre Oberfläche der Prothese ist derjenige Teil ihrer Oberfläche, der dazu bestimmt ist, im trochantären Teil des Knochens zu liegen. Dieser Oberflächenteil läßt sich auch an der noch nicht implantierten Prothese leicht bestimmen, weil man weiß, in welcher Weise sie zu implantieren ist und welche Lage sie demzufolge im Knochen normalerweise einnehmen wird.

In der Mitte des trochantären Teils des Knochens ist die Spongiosa mitunter weniger dicht ausgebildet, als nahe der Knochenrinde. Vorzugsweise befinden sich deshalb die nach ventral und dorsal gewendeten Anteile der trochantären, osteoinduktiv ausgerüsteten Oberflächenanteile der Prothese in einem gewissen Abstand von der Mitttelebene des Knochens. Deshalb soll der diese Oberflächenanteile bildende Teil der Prothese in AP-Richtung nicht zu dünn sein. Seine Dicke und damit der Abstand der genannten Oberflächenanteile liegt zweckmäßigerweise über 6 und weiter vorteilhaft über 9 mm bis etwa 15 mm.

Das Anwachsen frischer Knochenzellen an der Prothesenoberfläche kann durch einen Preßsitz der betreffenden Flächenanteile gefördert werden. Zweckmäßig ist es deshalb, die betreffenden Flächen und deren Gegenflächen in der Richtung, in welcher die Prothese in den Knochen eingefügt wird, keilförmig auszuführen und die der Prothese zugehörige Raspel, die dazu benutzt wird, den Aufnahmeraum für den Prothesenschaft zu formen, mit knapperen Querschnittsmaßen auszustatten, so daß beim Einschieben des Prothesenschafts in den durch die Raspel vorbereiteten Raum die betreffenden Flächenanteile Knochenmaterial verdrängen.

Die Erfindung wird im folgenden näher unter Bezugnahme auf die Zeichnung erläutert. Darin zeigen:
- Fig. 1: einen Längsschnitt durch den proximalen Abschnitt des Oberschenkelknochens in der Ebene, die die Längsachse des Henkelhaltes enthält,
- Fig. 2: einen Querschnitt gemäß der Schnittlinie II-II der Fig. 1 und
- Fig. 3: einen Schnitt durch die Prothese gemäß der Schnittlinie III-III der Fig. 1.

Mit durchgezogenen Linien ist in Fig. 1 der äußere Umriß des Knochens dargestellt. Auch die innere Grenzlinie der kompakten Knochenrinde 1 ist mit durchgezogenen Linien skizziert, soweit ihre Dicke beträchtlich ist. Dies ist im Bereich der Diaphyse 2 der Fall. In der Metaphyse 3 ist die Knochenrinde überwiegend so dünn, daß sie nur durch eine Linie angegeben wurde.

Die Metaphyse 3 und teilweise auch der obere Teil der Diaphyse ist mit nicht dargestellter, spongiöser Knochensubstanz gefüllt. Im vorliegenden Fall interessiert in dieser Hinsicht nur der dem größeren Trochanter zugeordnete, sogenannte trochantäre Bereich 4, der schraffiert dargestellt ist. Für die Zwecke der vorliegenden Erfindung wird er durch die Grenzlinien H und T definiert. Bei der Ersteren handelt es sich um die Mittellinie des Schenkelhalses 5 und des Hüftkopfs 6. Die letztere ist die Tangente an den Hüftkopf 6 vom Punkt S, in welchem sich die Linie H und die Mittelachse M der proximalen Diaphyse in der Zeichenebene schneiden. Zum trochantären Teil des Knochen gehören außerdem die vor und hinter der Zeichenebene im Bereich der schraffierten Fläche 4 befindlichen Knochenteile.

Die in den Knochen einzusetzende Prothese ist mit gestrichelten Linien in Fig. 1 und mit durchgezogenen Linien in Fig. 2 und 3 angedeutet. Sie besteht aus einem Kopf 10, einem Hals 11 und einem Schaft 12, von welchem ein Vorsprung 13 in den trochantären Bereich 4 des Knochen vorspringt. Der Vorsprung 13 ist mit Bohrungen 14 versehen, die nach ventral blickende Flächenbereiche bilden. Diese sind gegenüber der Lateralrichtung 19 hinterschnitten. Knochensubstanz, die in diese Bohrungen einwächst, faßt hinter die hinterschnittenen Flächen und trägt dadurch zur Übertragung von Zugkräften von der Prothese auf den trochantären Knochenbereich bei. Es versteht sich, daß der Vorsprung und die hinterschnittenen Flächen auch in anderer, bekannter Weise ausgebildet sein können. Beispielsweise kann der Vorsprung blatt- oder bügelförmig sein. Statt Bohrungen kann er größere Durchbrechungen oder Rippen zur Bildung hinterschnittener Flächen aufweisen. Diejenigen Teile der Prothese, die im Normalfall bei planmäßiger Implantation im trochantären Bereich 4 zu liegen bestimmt sind, werden als trochantäre Teile bzw. trochantäre Oberfläche der Prothese bezeichnet. Dazu gehört insbesondere der Vorsprung 13.

Dargestellt ist ein Prothesentyp, dessen Schaft 12 so ausgebildet und implantiert ist, daß er in der Diapyse 2 des Knochens fest verankert ist. Diese Art der Verankerung führt zu starker Entlastung des trochantären Knochenbereichs. Deshalb ist die Verwendung der Erfindung besonders für diesen Prothesentyp vorteilhaft. Sie kann aber auch bei anderen Prothesentypen Verwendung finden, die zu einem wesentlichen Teil in der Metaphyse verankert sind und/oder sich mittels eines (nicht dargestellten) Kragens auf der Resektionsfläche 15 des Knochens abstützen.

Die Oberfläche des Schafts 12 oder Teile davon können in bekannter Weise so gestaltet oder ausgerüstet sein, daß die Verbindung mit dem Knochen begünstigt wird. Beispielsweise kann eine Beschichtung vorgesehen sein, die porig ist und/oder aus Kalziumphosphat oder Hydroxylapatit besteht. Eine solche Schicht kann ganz oder teilweise auch im trochantären Bereich der Prothese vorgesehen sein. Beispielsweise ist in Fig. 1 durch Kreuzschraffur und in Fig. 2 und 3 durch Strichelung ein Bereich 16 angedeutet, der mit einer Beschichtung versehen ist. Diese Beschichtung enthält erfindungsgemäß eine osteoinduktive Substanz.

Die osteoinduktive Beschichtung in den entsprechenden Bereichen 16 führt zu intensivem Knochenwachstum im unmittelbaren Kontakt mit der Prothesenoberfläche und auch in etwaigen hinterschnittenen Bereichen. Daher können die auf die Prothese ausgeübten Kräfte wirksam von deren Oberfläche auf den trochantären Bereich 4 des Knochens übertragen werden. Er wird dadurch in den Kraftfluß eingebunden, und einem Knochenabbau wird vorgebeugt.

Fig. 2 veranschaulicht, daß der Vorsprung 13 eine beträchtliche Dicke in anterio-posteriorer Richtung aufweist. Seine anterioren und posterioren Oberflächenanteile 16 sind daher dem mittleren Bereich entrückt, in welchem die spongiose Knochensubstanz in manchen Fällen verarmt ist, und befinden sich in einem der Knochenrinde näheren und dichteren Bereich. Damit wird die Wahrscheinlichkeit einer guten Verbindung zwischen der Knochenoberfläche und der Knochensubstanz weiter vergrößert.

Fig. 3 veranschaulicht die Querschnittsform des Vorsprungs 13 in der Schnittrichtung III-III, die auch etwa der Einsetzrichtung entspricht. Wenn die Hohlform, die mittels einer Raspel zur Aufnahme der Prothese vorbereitet wurde, etwas knapper als die Prothesenform ist, ist mit dem Einfügen dieser Keilform in den Knochen eine Verdrängung von Knochensubstanz und dadurch eine Erhöhung der von der Knochensubstanz auf die Prothesenoberfläche ausgeübten Pressung verbunden. Auch dadurch wird ein rasch wachsender und inniger Verbund der Prothesenoberfläche mit dem Knochen gefördert.

Im dargestellten Beispiel ist lediglich die Beschichtung 16 des Vorsprungs 13 mit einer osteoinduktiven Substanz versehen. Die dadurch bewirkte innige Verbindung zwischen der Prothesenoberfläche und dem Knochen kann hinderlich sein im Falle einer Revision, weil die betreffenden Prothesenteile schwer aus dem Knochen ohne Beschädigung des Knochens entfernt werden können. Nach einem besonderen Merkmal der Erfindung ist deshalb der Vorsprung 13 vom Schaft 12 lösbar ausgebildet. Beispielsweise kann er längs einer Verbindungsfuge 17, die in Fig. 1 und 2 gestrichelt angedeutet ist, mittels Schrauben 18 oder anderer Verbindungsmittel mit dem Schaft 12 verbunden sein und von diesem gelöst werden, bevor der Schaft dem Knochen entnommen wird. Anschließend kann der Vorsprung leichter aus dem ihn umgebenden und mit ihm verwachsenen Knochen herausgelöst werden.

## Patentansprüche

1. Hüftgelenkprothese mit einem in den Oberschenkelknochen einzusetzenden Schaft (12), der einen im trochantären Bereich (4) zu liegen bestimmten trochantären Oberflächenteil aufweist, der eine das Knochenwachstum begünstigende Beschichtung (16) trägt, **dadurch gekennzeichnet, daß** ausschließlich dieser trochantäre Oberflächenteil oder ein Teil desselben eine osteoinduktive Substanz aufweist.

2. Hüftgelenkprothese nach Anspruch 1, **dadurch gekennzeichnet, daß** ausschließlich ein vom Schaft (12) innerhalb des trochantären Bereichs (4) vorspringender trochantärer Vorsprung (13) die osteoinduktive Substanz aufweist.

3. Hüftgelenkprothese nach Anspruch 1 oder 2, **dadurch gekennzeichnet, daß** die osteoinduktive Substanz von einer Beschichtung gebildet oder Teil einer Beschichtung ist.

4. Hüftgelenkprothese nach einem der Ansprüche 1 bis 3, **dadurch gekennzeichnet, daß** die osteoinduktive Substanz ein Bisphosphonat oder ein BMP umfaßt.

5. Hüftgelenkprothese nach einem der Ansprüche 1 bis 4, **dadurch gekennzeichnet, daß** mindestens der die osteoinduktive Substanz enthaltende Teil der trochantären Prothesenoberfläche porös ist.

6. Hüftgelenkprothese nach einem der Ansprüche 1 bis 5, **dadurch gekennzeichnet, daß** die osteoinduktive Substanz an Flächenanteilen der trochantären Prothesenoberfläche angebracht ist, die in bezug auf die Lateralrichtung (17) hinterschnitten sind.

7. Hüftgelenkprothese nach einem der Ansprüche 1 bis 6, **dadurch gekennzeichnet, daß** die trochantäre Prothesenoberfläche mindestens eine ventrale und eine dorsale Fläche umfaßt, die mit einer osteoinduktiven Beschichtung (16) versehen sind und einen Abstand in AP-Richtung von mehr als 6 mm, vorzugsweise mehr als 9 mm einschließen.

8. Hüftgelenkprothese nach Anspruch 2, **dadurch gekennzeichnet, daß** der Vorsprung (13) in Implantationsrichtung keilförmig ist und eine der Prothese zugeordnete Raspel im Bereich dieses Vorsprungs ein geringeres Volumen aufweist.

9. Hüftgelenkprothese nach Anspruch 2 oder 8, **dadurch gekennzeichnet, daß** der Vorsprung (13) vom Schaft lösbar ist.

## Claims

1. Hip-joint prosthesis with a stem (12) which is to be inserted into the femur and which has a trochanteric surface part intended to lie in the trochanteric region (4) and having a coating (16) that promotes growth of bone, **characterized in that** exclusively this trochanteric surface part or a part thereof comprises an osteoinductive substance.

2. Hip-joint prosthesis according to Claim 1, **characterized in that** the osteoinductive substance is provided exclusively on a trochanteric projection (13) protruding from the stem (12) within the trochanteric region (4).

3. Hip-joint prosthesis according to one of Claim 1 or 2, **characterized in that** the osteoinductive substance is formed by a coating or is part of a coating.

4. Hip-joint prosthesis according to one of Claims 1 to 3, **characterized in that** the osteoinductive substance includes a bisphosphonate or a BMP.

5. Hip-joint prosthesis according to one of Claims 1 to 4, **characterized in that** at least the part of the trochanteric prosthesis surface containing the osteoinductive substance is porous.

6. Hip-joint prosthesis according to one of Claims 1 to 5, **characterized in that** the osteoinductive substance is applied on surface portions of the trochanteric prosthesis surface which are undercut with respect to the lateral direction (17).

7. Hip-joint prosthesis according to one of Claims 1 to 6, **characterized in that** the trochanteric prosthesis surface comprises a ventral surface and a dorsal surface which are provided with an osteoinductive coating (16) and are at a distance from one another, in the AP direction, of more than 6 mm, preferably more than 9 mm.

8. Hip-joint prosthesis according to Claim 2, **characterized in that** the projection (13) is wedge-shaped in the direction of implantation, and a rasp assigned to the prosthesis has a smaller volume in the area of this projection.

9. Hip-joint prosthesis according to Claim 2 or 8, **characterized in that** the projection (13) can be detached from the stem.

## Revendications

1. Prothèse de la hanche munie d'une tige (12) à insérer dans le fémur, présentant une partie surfacique destinée à être placée au niveau du trochantère (4) et comportant un revêtement (16) favorisant la croissance osseuse, **caractérisée en ce qu'**exclusivement cette partie surfacique située au niveau du trochantère ou une partie de celle-ci contient une substance ostéoinductive.

2. Prothèse de la hanche selon la revendication 1, **caractérisée en ce qu'**exclusivement une saillie (13) du trochantère dépassant de la tige (12) à l'intérieur de la zone du trochantère (4) présente la substance ostéoinductive.

3. Prothèse de la hanche selon la revendication 1 ou 2, **caractérisée en ce que** la substance ostéoinductive est constituée par un revêtement ou fait partie d'un revêtement.

4. Prothèse de la hanche selon l'une quelconque des revendications 1 à 3, **caractérisée en ce que** la substance ostéoinductive contient un biphosphonate ou un BMP.

5. Prothèse de la hanche selon l'une quelconque des revendications 1 à 4, **caractérisée en ce qu'**au moins la partie contenant la substance ostéoinductive de la surface prothétique du trochantère est poreuse.

6. Prothèse de la hanche selon l'une quelconque des revendications 1 à 5, **caractérisée en ce que** la substance ostéoinductive est appliquée sur des sections surfaciques de la surface prothétique du trochantère qui est contre-dépouillée par rapport au sens latéral (17).

7. Prothèse de la hanche selon l'une quelconque des revendications 1 à 6, **caractérisée en ce que** la surface prothétique du trochantère comporte au moins une surface ventrale et une surface dorsale qui sont pourvues d'un revêtement ostéoinductif (16) et circonscrivent dans le sens AP une distance de plus de 6 mm, de préférence de plus de 9 mm.

8. Prothèse de la hanche selon la revendication 2, **caractérisée en ce que** la saillie (13) est en forme de coin dans le sens d'implantation et qu'une rugine associée à la prothèse présente un volume moindre au niveau de cette saillie.

9. Prothèse de la hanche selon l'une quelconque des revendications 2 ou 8, **caractérisée en ce que** la saillie (13) peut être détachée de la tige.
